# EUROPEAN PATENT APPLICATION

(11) **EP 4 533 994 A1**
(43) Date of publication of application: **09.04.2025**
(21) Application number: 23864654.1
(22) Date of filing: 11.09.2023
(51) Int. Cl.: A61B 1/00, A61B 1/12

(54) **PLACEMENT ASSEMBLY FOR ENDOSCOPE AND METHOD FOR USING SAME**

(30) Priority: 13.09.2022 CN 202211108995
(71) Applicant: NINGBO HITCM MEDICAL DEVICES CO., LTD., Ningbo, Zhejiang 315000 (CN)
(72) Inventor: DING, Weijiang, Ningbo, Zhejiang 315000 (CN); CAO, Xindong, Ningbo, Zhejiang 315000 (CN); YUAN, Peng, Ningbo, Zhejiang 315000 (CN)
(74) Representative: Viering, Jentschura & Partner mbB Patent- und Rechtsanwälte
(86) International application number: PCT/CN2023/117960
(87) International publication number: WO 2024/055922

(57) **Abstract**

Disclosed is a placement assembly for receiving an endoscope, which comprises: a tubular body extending along a longitudinal axis of the placement assembly and configured for receiving an endoscope; a proximal sealing member hermetically connected to a proximal end of the tubular body; a distal sealing member hermetically connected to a distal end of the tubular body; one or more fluid supply paths comprising a fluid input port for receiving fluid from a fluid source, a fluid discharge port for discharging the fluid received from the fluid source into a cavity, and a fluid pipeline allowing the fluid input port to be in fluid communication with the fluid discharge port; and one or more fluid control members disposed at positions corresponding to the corresponding fluid discharge ports. A method for cleaning the placement assembly is also disclosed.

## Description

For all purposes, the present application claims priority of the Chinese Patent Application No. 202211108995.4, filed with the CNIPA on September 13, 2022, the disclosure of the above-mentioned Chinese patent application is incorporated herein by reference in its entirety as part of the present application.

### TECHNICAL FIELD

The present disclosure relates to the field of medical instruments, particularly to an access assembly for receiving an endoscope, and more particularly to an access assembly for receiving an endoscope, which can automatically stop and start ejecting cleaning and/or drying fluid in response to the endoscope extending from and retracting into the distal end of the access assembly, respectively. In addition, the present disclosure also relates to a method of using the above-mentioned access assembly.

### BACKGROUND

Minimally invasive surgical procedures such as endoscopic surgery can reduce the invasiveness of surgical procedures. Endoscopic surgery involves surgery through the body wall, for example, to observe and/or operate on ovary, uterus, gallbladder, intestine, kidney, appendix, etc. There are many common endoscopic surgical procedures including, for example, arthroscopy, laparoscopy, gastroenteroscopy, and laryngobronchoscopy. In these methods, a puncture cone passing through an access assembly is used to create an incision on the patient's body surface which is opened to a target site, and the endoscopic surgery is performed through the incision. After the puncture is formed, the access assembly extends, through the incision, into the body cavity and remains in the body cavity while the penetration cone is withdrawn from the access assembly to provide an access for endoscopic surgical instruments. A camera or endoscope is inserted through the access assembly to allow for visual inspection and magnification of the body cavity. The surgeon can then perform diagnosis and/or treatment at the surgical site with the aid of specialized instruments (such as forceps, graspers, cutters, applicators, etc.) designed to be fitted through an additional cannula.

During use, the lens of an endoscope may become covered with condensation, tissue, blood and other body fluids, etc. in the body cavity. It is therefore difficult to keep the lens of an endoscope clean during the surgery. Conventionally, a surgeon (such as an endoscopist) withdraws the endoscope from the incision on the patient's body via the access assembly, cleans the lens with pre-prepared saline at body temperature, and then wipes it with a disinfectant such as iodophor and re-inserts it into the incision on the patient's body via the access assembly. During the surgical procedure, the time required to clean the lens may increase the total time of the surgery and the amount of time the patient needs to remain anesthetized; and since the endoscope is repeatedly withdrawn from and inserted into the incision on the patient's body, this may result in increased risk of infection and increase the recovery time. Although there are currently some access assemblies that can flush the lens of an endoscope with liquid in the body cavity, they are not accepted by most surgeons and have not been promoted and applied because these access assemblies directly discharge the flushing liquids and condensate, tissue, blood and other body fluids into the patient's body cavity. **In** addition, operations of starting and stopping the ejection of fluid usually require for setting up respective valves in the pipeline or frequently operating pump switches, thereby increasing the complexity of the access assembly system for endoscope and making the operation cumbersome. It can be seen that there is a need in the art for an improved access assembly which enables easy and rapid cleaning of the lens of an endoscope during surgery.

### SUMMARY

The present disclosure relates to an access assembly for receiving an endoscope. The access assembly may include a tubular body, a proximal seal member, a distal seal member, one or more fluid supply passages, and one or more fluid control components. The tubular body may be configured to extend along a longitudinal axis of the access assembly and to receive an endoscope. The proximal seal member may be configured to be connected to a proximal end of the tubular body in a sealed manner. The distal seal member may be configured to be connected to a distal end of the tubular body in a sealed manner. An inner wall of the tubular body, a distal surface of the proximal seal member, and a proximal surface of the distal seal member collectively define a cavity of the access assembly as an interior space enclosed by the access assembly. In some embodiments, the proximal seal member may be configured to form a seal against a side wall of the endoscope when the endoscope is inserted into the access assembly through the proximal seal member, so as to prevent substances external to the access assembly from entering the cavity of the access assembly. In some embodiments, the distal seal member may be configured to form a seal by closing a shape and a structure of the distal seal member when the endoscope does not pass through the proximal seal member, so as to prevent substances within the cavity of the access assembly from flowing out of the distal end of the access assembly. In some embodiments, the distal seal member may also be configured to form a seal against the side wall of the endoscope when the endoscope extends (e.g., extending into the body cavity of a patient) from the distal end of the access assembly through the distal seal member, so as to prevent substances within the cavity of the access assembly from flowing out of the distal end of the access assembly.

The one or more fluid supply passages may include a fluid input port for receiving a fluid from a fluid source, a fluid discharge port for discharging the fluid received from the fluid source into the cavity, and a fluid pipeline for fluidly communicating the fluid input port with the fluid discharge port. The one or more fluid control components may be arranged at a position corresponding to a respective fluid discharge port. Here, it should be understood that the corresponding position means that the fluid control component arranged at this position can produce corresponding blocking and unblocking effects on the respective fluid discharge port, as will be described in more detail below with reference to the accompanying drawings. When the distal end of the endoscope passes through the distal seal member and distally extends from the cavity, the fluid discharge port is clamped between the respective fluid control component and the side wall of the endoscope so as to be blocked. When the distal end of the endoscope is proximally retracted into the cavity through the distal seal member, the fluid discharge port is released from the respective fluid control component so as to restore the respective fluid discharge port to be unblocked. In response to the extension and retraction of the endoscope through the distal seal member, the ejection of fluid can be automatically started and stopped through the automatic clamping and release of the respective fluid discharge port by the fluid control component without operating any switch of a pump or any valve, thus simplifying the complexity and respective operation of the access assembly system.

In some embodiments, the one or more fluid control components may include one or more protrusions arranged at axial and circumferential positions corresponding to the respective fluid discharge ports, on an inner wall of the tubular body. Here, it should be understood that a corresponding axial and circumferential position means that the protrusion arranged at this position can produce corresponding blocking and unblocking effects on the respective fluid discharge port, as will be described in more detail below with reference to the accompanying drawings.

In some embodiments, the one or more protrusions may include at least one annular protrusion, which may be circumferentially arranged on the inner wall of the tubular body, at an axial position corresponding to the fluid discharge port. Here, it should be understood that the corresponding axial position means that the annular protrusion arranged at this position can produce corresponding blocking and unblocking effects on the respective fluid discharge port (for example, all of a plurality of fluid discharge ports), as will be described in more detail below with reference to the accompanying drawings.

In some embodiments, each of the one or more protrusions may not be arranged on the inner wall of the tubular body but on an outer surface of the respective fluid discharge port. In the case where the fluid control component is formed separately from, for example, the fluid pipeline on the inner wall of the tubular body, the one or more protrusions formed on the outer surface of the respective fluid discharge port and used as the fluid control components may not be considered to be aligned with the circumferential direction of the fluid discharge port.

In some embodiments, any two or more selected from the group consisted of the one or more fluid control components, the one or more fluid discharge ports, and the distal seal member are integrally formed from a flexible material. **In** this case, the installation process of the assembly is simplified and the alignment between the fluid discharge port and the fluid control component is facilitated.

In some embodiments, the flexible material may be a medical grade silicone, so as to facilitate the opening and closing of the distal seal member and to promote the fluid discharge port to be blocked when being clamped by the fluid control component and the inner wall of the tubular body of the access assembly and to be restored to be unblocked when being released from the above-mentioned clamping.

In some embodiments, the one or more fluid supply passages may include a plurality of fluid supply passages, which may provide cleaning liquid, gas for at least partially drying the endoscope or gas for producing artificial pneumoperitoneum, and a gas-liquid mixture that improves the cleaning effect through the impact of gas-liquid two-phase flow, respectively.

In some embodiments, the access assembly may also include a vacuum suction member for sucking fluid from the cavity to remove the above-mentioned liquid, gas or gas-liquid mixture from the cavity of the access assembly after cleaning and/or drying the endoscope.

In some embodiments, the fluid discharge port may be configured to be pointed in a proximal direction along the longitudinal axis of the access assembly, as will be described further below with reference to the accompanying drawings. In this case, fluid ejected from the fluid discharge port can be directed towards the lens at the distal end of the endoscope thereby improving cleaning effectiveness, and this fluid will not be affected by the orientation of the endoscope and the access assembly relative to gravity.

The present disclosure also relates to a method of using the access assembly described above, wherein an access assembly according to any one of the above embodiments is provided and an endoscope is inserted through the access assembly. The operation may include connecting the fluid input port to the fluid source and retracting the distal end of the endoscope proximally into the cavity through the distal seal member, so that the fluid discharge port previously clamped by the fluid control component and the inner wall of the tubular body of the access assembly at both sides is restored to be unblocked, so that the fluid is ejected from the fluid discharge port, thereby cleaning the distal end of the endoscope.

In some embodiments, the method may further include extending the distal end of the endoscope distally from the cavity through the distal seal member, so that the fluid discharge port is blocked by being clamped by the fluid control component and the inner wall of the tubular body of the access assembly at both sides, so as to stop the fluid from being discharged into the cavity of the access assembly.

In some embodiments, the fluid source provides one of a liquid, a gas, or a gas-liquid mixture.

It should be understood that the above method of cleaning the lens of an endoscope can be used not only during surgery, but also during operations such as equipment acceptance, maintenance and test that do not involve patients, to provide easy and fast cleaning of the lens of an endoscope.

### BRIEF DESCRIPTION OF DRAWINGS

An access assembly for endoscope and an operating method of the access assembly disclosed in the present disclosure are described below with reference to the accompanying drawings. It should be understood that the drawings are for illustration and explanation purposes only, and are not intended to limit the scope of protection of the present disclosure in any way. In addition, each drawing only schematically shows the position and combination relationship of various components and is not necessarily drawn to scale, wherein:
Fig. 1 is a cross-sectional side view of an access assembly for endoscope according to an embodiment of the present disclosure;
Fig. 2A is a partially enlarged cross-sectional side view of a distal end of the access assembly for endoscope in Fig. 1 in a state of the endoscope extending from the distal end;
Fig. 2B is a partially enlarged cross-sectional side view of the distal end of the access assembly for the endoscope in Fig. 1 in a state of the endoscope being retracted into the cavity; and
Figs. 3A-3C are respectively a partially enlarged bottom side perspective view, a partially enlarged cross-sectional side view, and a partially enlarged top side perspective view of a distal end of an access assembly for endoscope according to an embodiment of the present disclosure.

### DETAILED DESCRIPTION

An access assembly capable of cleaning a lens of an endoscope during a surgery is described in detail with reference to the accompanying drawings, wherein the same reference numerals refer to the same or corresponding elements in each of the several views. As used herein, the term "distal" refers to the direction (e.g., the downward direction as shown generally in the drawings) in which the endoscope, part of the access assembly or components thereof are far away from the operator (e.g. doctor), for example, "distal direction/distally" refers to the direction in which the endoscope is inserted, and "distal end" refers to one end of the direction in which the endoscope is inserted; and the term "proximal" refers to the direction (e.g., the upward direction shown generally in the drawings) in which the endoscope, part of the access assembly or components thereof are close to the operator, for example, "proximal direction/proximally" refers to the direction in which the endoscope is withdrawn, and "proximal end" refers to one end of the direction in which the endoscope is withdrawn. Additionally, the term "endoscope" is generally used interchangeably with laparoscope, arthroscope, gastroenteroscope, laryngobronchoscope, or any other device used for observing a patient's body cavity through an incision or cannula with small diameter. As used herein, the term "fluid" generally refers to a substance with fluidity, including but not limited to liquids (such as pure liquids, solutions, colloids, suspensions, turbid liquid), gases, and gas-liquid two-phase flow mixtures, plasmas, fluidized solid particles, etc. As used herein, the term "about" means that the value is approximate and that small changes of the value will not significantly affect the practice of the disclosed aspects of the present disclosure. Where a numerical limitation is used, "about" means that the value may vary by ±10% and still be within the scope of the present disclosure, unless the context indicates otherwise.

By using the access assembly for endoscope according to the present disclosure, the endoscope can be cleaned and dried during surgery or during operations such as equipment acceptance, maintenance and test without the need of taking the endoscope out of the access assembly as a whole, and the ejection of fluid can be started and stopped conveniently and automatically in response to the retraction and extension of the endoscope through the distal end of the access assembly without the need of operating the switches of a pump of the fluid source or operating the respective valves on the pipeline, thereby simplifying the operation flow and saving the cleaning fluid.

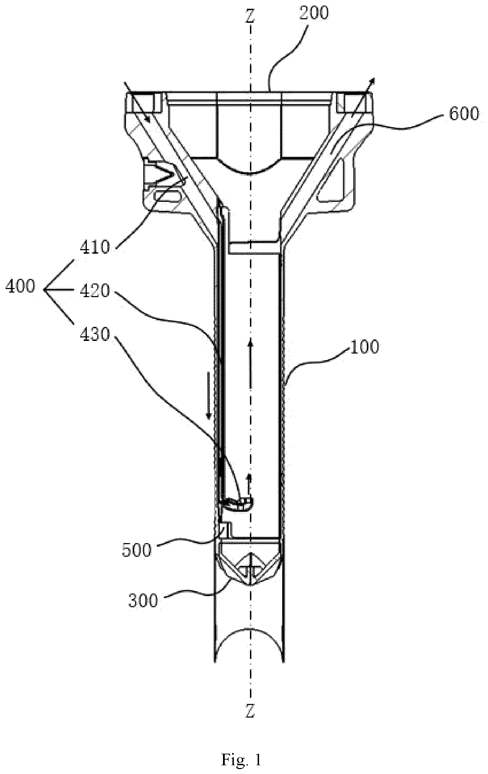

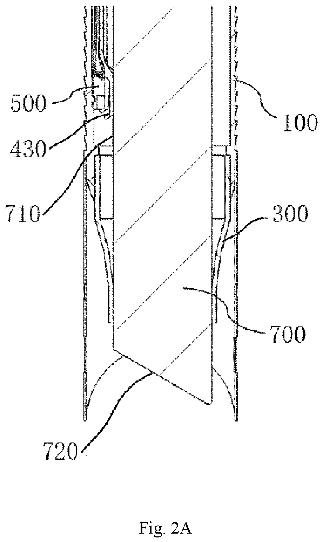

Fig. 1 is a cross-sectional side view of an access assembly for endoscope in accordance with an embodiment of the present disclosure. In some embodiments, the access assembly may include a tubular body 100, a proximal seal member 200, a distal seal member 300, one or more fluid supply passages 400, and one or more fluid control components 500. The tubular body 100 may be configured to extend along a longitudinal axis Z-Z' of the access assembly and to receive an endoscope (e.g., the endoscope 700 in Figs. 2A-2B). The proximal seal member 200 can be configured to be connected to the proximal end of the tubular body 100 in a sealed manner, and the distal seal member 300 can be configured to be connected to the distal end of the tubular body 100 in a sealed manner. An inner wall of the tubular body 100, a distal surface of the proximal seal member 200, and a proximal surface of the distal seal member 300 collectively define a cavity of the access assembly, and the cavity is an internal space enclosed by the access assembly.

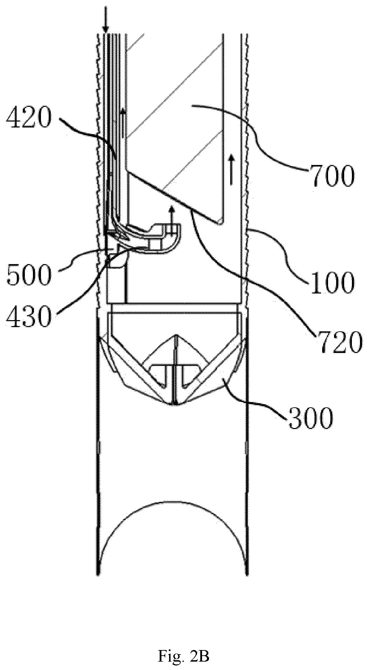

In some embodiments, the proximal seal member 200 may be configured to form a seal against a side wall of an endoscope (e.g., the side wall 710 of the endoscope 700 in Figs. 2A-2B) when the endoscope (e.g., the endoscope 700 in Figs. 2A-2B) is inserted into the cavity of the access assembly through the proximal seal member 200, so as to prevent substances outside the access assembly from entering the cavity of the access assembly. In some embodiments, the distal seal member 300 may be configured to be closed through its inherent shape and structure to form a seal when the endoscope does not pass through the distal seal member 300, thereby preventing substances within the cavity of the access assembly from flowing out of the distal end of the access assembly, as will be further described below with reference to Fig. 2B. In some embodiments, the distal seal member may also be configured to form a seal against the side wall of the endoscope when the endoscope extends (e.g., extending into the body cavity of a patient) from the distal end of the access assembly through the distal seal member to prevent substances within the cavity of the access assembly from flowing out of the distal end of the access assembly, as will be further described below with reference to Fig. 2A.

Each of the one or more fluid supply passages 400 may include a fluid input port 410 for receiving a fluid from a fluid source (not shown), a fluid discharge port 430 for discharging the fluid received from the fluid source into the cavity, and a fluid pipeline 420 for fluidly communicating the fluid input port 410 with the fluid discharge port 430. In some embodiments, the one or more fluid supply passages 400 may include a plurality of fluid supply passages 400 for providing one of cleaning liquid, drying gas, and a gas-liquid mixture, respectively. In some embodiments, the fluid discharge port 430 may be separately arranged on the inner wall of the tubular body 100 and connected to a distal end of the fluid pipeline 420. In some other embodiments, the fluid discharge port 430 may also be formed integrally with the distal seal member 300 (not shown).

Each of the one or more fluid control components 500 may be arranged at a position corresponding to a respective fluid discharge port 430. It should be understood that the position correspondence between the fluid control component 500 and the fluid discharge port 430 may include an alignment between their axial positions and an alignment between their circumferential positions. In particular, the corresponding position means that the fluid control component 500 arranged at this position can cooperate with the side wall of the endoscope (for example, the side wall 710 of the endoscope 700 in Fig. 2A) to block the respective fluid discharge port 430 and to restore the respective fluid discharge port 430 to be unblocked, respectively. Fig. 2A is a partially enlarged cross-sectional side view of a distal end of the access assembly for an endoscope 700 in Fig. 1 in a state of the endoscope 700 being extended from the distal end of the access assembly. The state shown in Fig. 2A generally corresponds to the state where the lens at the distal end 720 of the endoscope 700 is used to observe the body cavity of the patient during the surgery, or corresponds to an operation during the equipment acceptance, maintenance and test that does not involve the patient. It can be understood that since the lens is not within the cavity of the access assembly, the fluid is not expected to be ejected from the fluid discharge port 430 in this state. As shown in Fig. 2A, when the distal end 720 of the endoscope 700 passes through the distal seal member 300 and extends distally from the cavity of the access assembly, the fluid control component 500, along with the respective fluid discharge port 430, is pushed towards the inner wall of the tubular body 100 by the sidewall 710 of the endoscope 700. At this time, the fluid discharge port 430 is clamped between the respective fluid control component 500 and the side wall 710 of the endoscope 700, so that the respective fluid discharge port 430 is blocked to automatically stop the ejection of fluid.

Fig. 2B is a partially enlarged cross-sectional side view of a distal end of the access assembly for the endoscope 700 in Fig. 1 in a state of the endoscope 700 being retracted into the cavity of the access assembly. The state shown in Fig. 2B generally corresponds to the state where the lens at the distal end 720 of the endoscope 700 is retracted into the cavity of the access assembly for cleaning during the surgery, or corresponds to an operation during equipment acceptance, maintenance and test that does not involve patients. It can be understood that in this state, the cleaning liquid or drying gas or gas-liquid mixture is expected to be ejected from the fluid discharge port 430 into the cavity of the access assembly to clean or at least partially dry the lens. As shown in Fig. 2B, when the distal end 720 of the endoscope 700 is proximally retracted into the cavity of the access assembly through the distal seal member 300, the side wall 710 of the endoscope 700 no longer pushes the fluid discharge port 430 towards the inner wall of the tubular body 100. At this time, the clamping of the fluid discharge port 430 by the side wall 710 of the endoscope 700 and the fluid control component 500 is released, and the fluid discharge port 430 returns to its original shape under its own elastic force and is restored to be unblocked, thereby starting to eject fluid under the action of the fluid pressure from the fluid source. When the fluid is ejected, the general flowing direction of the fluid in the access assembly is shown by the arrows in Fig. 2B.

In response to the extension and retraction of the endoscope 700 through the distal seal member 300, the respective fluid discharge port 430 is automatically clamped and released by the fluid control component 500 in cooperation with the side wall 710 of the endoscope 700, so that the access assembly for the endoscope 700 according to the present disclosure can automatically realize starting and stopping the ejection of fluid without operating any switch of a pump or any valve, thereby simplifying the complexity and respective operation of the access assembly system.

In some embodiments, the access assembly for endoscope may also include a vacuum suction member 600. The vacuum suction member 600 may be connected to a vacuum suction source (such as a vacuum pump, a vacuum interface as a facility in surgical room, etc.) to discharge the fluid (e.g., cleaning liquid, drying gas, gas-liquid mixture) from the cavity of the access assembly. During cleaning and drying operations, the general flowing direction of the fluid is shown by the arrows in Fig. 1.

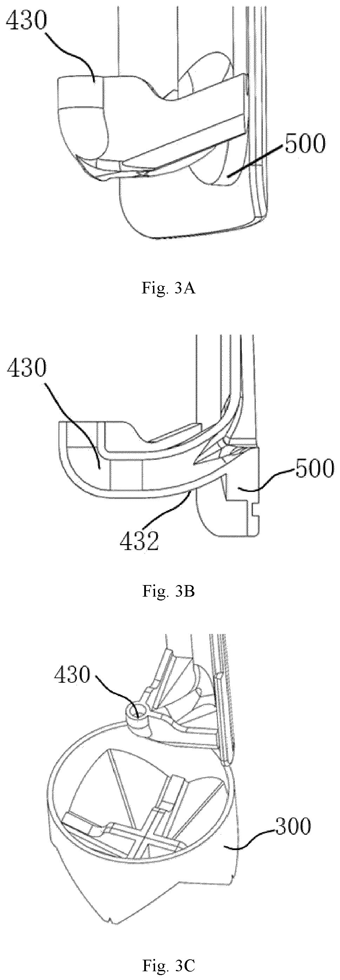

Fig. 3A-3C are a partially enlarged bottom side perspective view, a partially enlarged cross-sectional side view, and a partially enlarged top side perspective view of a distal end of an access assembly for endoscope according to an embodiment of the present disclosure, respectively. In some embodiments, any two or more selected from the group consisted of the one or more fluid control components 500, the fluid discharge port 430 of each fluid supply passage 400 of the one or more fluid supply passages 400, and the distal seal member 300 can be integrally formed from a flexible material, as shown in Figs. 3A-3C. In this case, the installation process of the assembly is simplified and the alignment between the fluid discharge port 430 and the fluid control component 500 is facilitated. In some embodiments, the flexible material may be a medical grade silicone to facilitate the opening and closing of the distal seal member 300, and to facilitate the fluid discharge port 430 to be blocked when it is clamped by the fluid control component 500 and the inner wall of the tubular body 100 of the access assembly and to be elastically restored to be unblocked when it is released from the above clamping.

In some embodiments, as shown in Figs. 3A and 3B, the fluid discharge port 430 may have an opening generally orientated towards a proximal side of the access assembly to eject the fluid to the distal end 720 of the endoscope 700. In some embodiments, as shown in Figs. 3A and 3B, the fluid control component 500 may be located radially outside the fluid discharge port 430, and located at an axial position substantially corresponding to the fluid discharge port 430 along the longitudinal axis Z-Z', so as to cooperate with the side wall 710 of the endoscope 700 to clamp and release the fluid discharge port 430, as described in more detail with reference to Figs. 2A and 2B. For example, in a state where the fluid discharge port 430 is pushed towards the inner wall of the tubular body 100 by the side wall 710 of the endoscope 700, the fluid control component 500 may be located at an axial and circumferential position corresponding to a middle section of the fluid discharge port 430 and radially close to the inner wall of the tubular body 100.

In some embodiments, the distal seal member 300 may include a plurality of sealing valves, as shown in Fig. 3C. When the endoscope 700 does not pass through the distal seal member 300, the plurality of sealing valves can be elastically closed by virtue of their own shapes and form a seal. As the endoscope 700 passes through the distal seal member 300, the plurality of sealing valves may be elastically expanded, gradually, against the endoscope 700 and remain sealed against the sidewall 710 of the endoscope 700, thereby preventing substances within the cavity of the access assembly from flowing out of the distal end of the access assembly.

Although Figs. 3A-3C illustrate the fluid discharge port 430 as a part separate from the distal seal member 300, in some other embodiments, the fluid discharge port 430 may also be located on the distal seal member 300, for example, on a proximal surface of at least one sealing valve of the distal seal member 300. Accordingly, the fluid control component 500 may, similarly, cooperate with the sidewall 710 of the endoscope 700 to clamp and release the fluid discharge port 430. Additionally, although the fluid discharge port 430 is shown in the drawings as being located close to the distal end of the tubular body 100 along the longitudinal axis Z-Z', in some other embodiments, the fluid discharge port 430 and the corresponding fluid control component 500 may also be located at a position in the middle of the tubular body 100 or close to the proximal end of the tubular body 100 along the longitudinal axis Z-Z'. In this case, the automatic clamping and releasing of the fluid discharge port 430 can still be achieved merely by correspondingly adjusting a distance that the distal end 720 of the endoscope 700 retracts along the longitudinal axis Z-Z' during operation.

In some embodiments, the one or more fluid control components 500 may include one or more protrusions, and the number of the one or more protrusions may be equal to the number of the one or more fluid supply passages 400 and the number of the respective one or more fluid discharge ports 430. It will be appreciated that in the case where the access assembly includes a plurality of fluid supply passages 400 and a plurality of respective fluid discharge ports 430, the respective fluid input port 410 corresponding to each fluid supply passage 400 may be connected to a different fluid source, such as a cleaning liquid source, a drying gas source or a gas-liquid mixture source, so that the access assembly can eject different fluids into the cavity of the access assembly as required. Each protrusion may be arranged on the inner wall of the tubular body 100, at an axial and circumferential position corresponding to the respective fluid discharge port 430. In this case, with the endoscope 700 extending through the distal seal member 300, the shape of the protrusion of the fluid control component 500 may cooperate with the side wall 710 of the endoscope 700 to promote the clamping of the respective fluid discharge port 430, thereby stopping the ejection of fluid.

In some embodiments, the access assembly may include a plurality of fluid supply passages 400, and each fluid supply passage 400 may have a separate fluid input port 410, a separate fluid discharge port 430, and a separate fluid pipeline 420. In some embodiments, the respective fluid input ports 410 of the plurality of fluid supply passages 400 can be respectively connected to fluid sources that supply different fluids (e.g., cleaning liquid, drying gas, gas-liquid mixture, etc.), thereby satisfying various functions of cleaning and at least partially drying the lens of the endoscope 700. It should be understood that, during the cleaning and optionally the drying process as described in more detail below, it may be necessary for the fluid supply system (not shown) to automatically or manually stop supplying liquid, and to be switched to supplying optional drying gas after the cleaning effect satisfies the cleanliness requirements, but the access assembly according to the present disclosure can still automatically stop the ejection of drying gas after the drying effect is achieved to a desired degree. More generally, during a multi-step cleaning process of the endoscope 700 (for example, including cleaning, drying and other steps), the fluid control component 500 cooperates with the side wall 710 of the endoscope 700 to automatically clamp and release the fluid discharge port 430 as the distal end 720 of the endoscope 700 extends distally and retracts proximally, and the access assembly according to the present disclosure can at least automatically start the ejection of fluid (inflow, cleaning liquid, gas-liquid mixture two-phase flow, etc.) in the first step and automatically stop the ejection of fluid (for example, drying gas) in the last step.

Furthermore, in some other embodiments, at least some of the respective fluid input ports 410 of the plurality of fluid supply passages 400 may be connected to the same fluid source (e.g., cleaning liquid, drying gas, gas-liquid mixture, etc.), but the respective fluid discharge ports 430 of the plurality of fluid supply passages 400 may be arranged differently in positions. For example, a plurality of fluid discharge ports 430 may be distributed at different radial, axial and/or circumferential positions around the longitudinal axis Z-Z' so that the ejection of fluid can be distributed on the lens of the endoscope 700 in a better way, thereby improving the cleaning and drying effects.

In some embodiments, the one or more protrusions that are used as the fluid control components 500 may include an annular protrusion (not shown), which may be circumferentially close to the inner wall of the tubular body 100 and located at an axial position corresponding to the fluid discharge port 430. In some embodiments, the annular protrusion may be in a closed or non-closed annular shape, such as a half ring. It can be understood that in an embodiment (not shown) in which the access assembly has a plurality of fluid supply passages 400 and a plurality of respective fluid discharge ports 430 (for example, the plurality of fluid supply passages 400 are used for discharging cleaning liquid, drying gas and gas-liquid mixture, respectively), one annular protrusion can cooperate with the side wall 710 of the endoscope 700 to achieve the clamping and the release of all the plurality of fluid discharge ports 430 at the same time, and it is not necessary to consider the circumferential alignment with the plurality of fluid discharge ports 430, respectively, when installed to the tubular body 100. In some embodiments, the corresponding axial position means that the annular protrusion arranged at this position can produce corresponding blocking and unblocking effects on the respective fluid discharge port 430 (for example, all the plurality of fluid discharge ports 430). For example, the axial position of the annular protrusion along the longitudinal axis Z-Z' may generally correspond to the axial position of the middle section of the fluid discharge port 430 in a state where the fluid discharge port 430 is pushed towards the inner wall of the tubular body 100 by the side wall 710 of the endoscope 700.

In some embodiments, each of the one or more protrusions may not be arranged close to the the inner wall of the tubular body 100 but may be arranged on the outer surface 432 (not shown) of the respective fluid discharge port 430. It can be understood that in the case where the fluid control component 500 is formed separately from, for example, the fluid pipeline 420 on the inner wall of the tubular body 100, one or more protrusions serving as the fluid control component 500 will be formed on the outer surface 432 of the respective fluid discharge port 430, so that the above-mentioned clamping and releasing effects can be achieved similarly, and the circumferential alignment with the fluid discharge port 430 does not need to be considered during installation.

In some embodiments, the fluid discharge port 430 may be arranged to be directed to a proximal direction (i.e., the generally upward direction in the drawings) along the longitudinal axis of the access assembly (Z-Z' in Fig. 1), as shown in Figs. 3A-3C. In this case, the fluid ejected from the fluid discharge port 430 can be directed to the lens at the distal end 720 of the endoscope 700 when the endoscope 700 is retracted into the access assembly, thereby improving the cleaning effect, and this fluid will not be affected by the orientation of the endoscope 700 and the access assembly relative to gravity. Alternatively, in some other embodiments, the fluid discharge port 430 may be arranged along other orientations, for example, it may be partially radially inwardly oriented.

A method of using the above-mentioned access assembly is described below with reference to Figs. 2A and 2B. The access assembly according to the present disclosure can be operated by using the method according to the present disclosure to clean and dry the endoscope, in both of the case where the visual field of the endoscope is obscured by pollutants during the surgery and the case of an operation such as equipment acceptance, maintenance and test that does not involve patients.

In some embodiments, the method according to the present disclosure may include connecting respective fluid input ports 410 of one or more fluid supply passages 400 to respective fluid sources. In this method, an access assembly from any of the embodiments described above is provided and an endoscope 700 is inserted through the access assembly. As mentioned above, the fluid source may include a cleaning liquid source, a drying gas source, a gas-liquid mixture source, etc., to achieve different purposes such as cleaning and drying. When it is desired to discharge the fluid from the fluid discharge port 430 to clean or dry the lens at the distal end 720 of the endoscope 700, the distal end 720 of the endoscope 700 is proximally retracted into the cavity through the distal seal member 300, so that the fluid discharge port 430 previously clamped by the fluid control component 500 and the inner wall of the tubular body 100 of the access assembly at both sides is restored to be unblocked, so that the fluid is ejected from the fluid discharge port 430, thereby cleaning the distal end 720 of the endoscope 700.

The method according to the present disclosure may further include: after completing the cleaning and/or drying of the lens of the endoscope 700, extending the distal end 720 of the endoscope 700 distally from the cavity through the distal seal member 300 (for example, it is desired to continue to use the endoscope 700 to observe the body cavity of the patient), so that the fluid discharge port 430 is clamped by the fluid control component 500 and the inner wall of the tubular body 100 of the access assembly at both sides to be blocked, thereby stopping the fluid from being discharged into the cavity of the access assembly.

In some embodiments, each fluid source provides one of a liquid, a gas and a gas-liquid mixture to clean and dry the lens at the distal end 720 of the endoscope 700.

It will be understood that various modifications may be made to the disclosed methods and apparatus. Accordingly, the above description is not to be construed as limitative, but merely exemplary of aspects of the present disclosure. Those skilled in the art will envision other modifications within the scope and spirit of the present disclosure. For example, any and all features of one described aspect may be appropriately integrated into another aspect.

## Claims

1. An access assembly for receiving an endoscope, comprising:
a tubular body, configured to extend along a longitudinal axis of the access assembly and to receive the endoscope;
a proximal seal member, configured to be connected to a proximal end of the tubular body in a sealed manner;
a distal seal member, configured to be connected to a distal end of the tubular body in a sealed manner, wherein an inner wall of the tubular body, a distal surface of the proximal seal member, and a proximal surface of the distal seal member collectively define a cavity of the access assembly;
one or more fluid supply passages, comprising a fluid input port for receiving a fluid from a fluid source, a fluid discharge port for discharging the fluid received from the fluid source into the cavity, and a fluid pipeline for fluidly communicating the fluid input port with the fluid discharge port; and
one or more fluid control components, arranged at a position corresponding to a respective fluid discharge port, wherein
when a distal end of the endoscope extends distally from the cavity through the distal seal member, the fluid discharge port is clamped between a respective fluid control component and a side wall of the endoscope so that the fluid discharge port is blocked, and
when the distal end of the endoscope is retracted proximally into the cavity through the distal seal member, the fluid discharge port is released from a clamping between the respective fluid control component and the side wall of the endoscope so that the fluid discharge port is restored to be unblocked.

2. The access assembly according to claim 1, wherein the one or more fluid control components comprise one or more protrusions each arranged on the inner wall of the tubular body, at an axial and circumferential position corresponding to the respective fluid discharge port.

3. The access assembly according to claim 2, wherein the one or more protrusions comprise at least one annular protrusion circumferentially arranged on the inner wall of the tubular body, at an axial position corresponding to the fluid discharge port.

4. The access assembly according to claim 1, wherein the one or more fluid control components comprise one or more protrusions arranged on an outer surface of the respective fluid discharge port.

5. The access assembly according to any one of claims 1-4, wherein any two or more selected from the group consisted of the one or more fluid control components, the one or more fluid discharge ports, and the distal seal member are integrally formed from a flexible material.

6. The access assembly according to claim 5, wherein the flexible material is a medical grade silicone.

7. The access assembly according to claim 1, further comprising a vacuum suction member for sucking the fluid from the cavity.

8. The access assembly according to claim 1, wherein the fluid discharge port is configured to be directed to a proximal direction along the longitudinal axis of the access assembly.

9. A method of using an access assembly for receiving an endoscope, comprising
providing the access assembly according to any one of claims 1-8, and inserting the endoscope through the access assembly;
connecting the fluid supply passage to a respective fluid source; and
retracting the distal end of the endoscope proximally into the cavity through the distal seal member so that the fluid discharge port is restored to be unblocked to clean the distal end of the endoscope with the fluid.

10. The method according to claim 9, further comprising:
extending the distal end of the endoscope distally from the cavity through the distal seal member so that the fluid discharge port is blocked to stop discharging the fluid into the cavity.

11. The method according to claim 9, wherein the respective fluid source respectively provides one selected from the group consisted of a liquid, a gas, and a gas-liquid mixture.
